# EUROPEAN PATENT APPLICATION

(11) **EP 4 227 975 A1**
(43) Date of publication of application: **16.08.2023**
(21) Application number: 21917534.6
(22) Date of filing: 14.09.2021
(51) Int. Cl.: H01J 35/08, H05G 1/52, A61B 6/00

(54) **X-RAY GENERATION TARGET, X-RAY GENERATOR, AND X-RAY IMAGING SYSTEM**

(30) Priority: 05.01.2021 JP 2021000432
(71) Applicant: Hamamatsu Photonics K.K., Hamamatsu-shi, Shizuoka 435-8558 (JP)
(72) Inventor: FURUKI Hiroki, Hamamatsu-shi, Shizuoka 435-8558 (JP); KAWAKAMI Hiroki, Hamamatsu-shi, Shizuoka 435-8558 (JP)
(74) Representative: Grünecker Patent- und Rechtsanwälte PartG mbB
(86) International application number: PCT/JP2021/033743
(87) International publication number: WO 2022/149309

(57) **Abstract**

An X-ray generation target includes a plurality of long target parts generating X-rays in response to incidence of an electron beam and a target part holder in which the plurality of target parts are buried to be parallel to each other. The target part holder includes a mark part indicating an arrangement state of the target parts.

## Description

### Technical Field

The present disclosure relates to an X-ray generation target, an X-ray generator, and an X-ray imaging system.

### Background Art

An X-ray generator including an electron gun emitting an electron beam and an X-ray generation target including a plurality of target parts generating X-rays in response to incidence of an electron beam is known (for example, see Patent Literature 1). Such an X-ray generator is mounted, for example, in an X-ray imaging system.

### Citation List

### Patent Literature

[Patent Literature 1] Japanese Unexamined Patent Publication No. 2016-537797

### Summary of Invention

### Technical Problem

In the aforementioned technique, a plurality of target parts may be long and be arranged to be parallel to each other. In this case, when an arrangement state of the target parts is not a desired arrangement state, it may be difficult to acquire X-rays satisfying desired irradiation conditions.

The present disclosure has been invented in consideration of the aforementioned circumstances and provides an X-ray generation target, an X-ray generator, and an X-ray imaging system in which an arrangement state of a plurality of target parts in the X-ray generator can be set to a desired arrangement state.

### Solution to Problem

An X-ray generation target according to an aspect of the present disclosure includes a plurality of long target parts generating X-rays in response to incidence of an electron beam and a target part holder in which the plurality of target parts are buried to be parallel to each other, and the target part holder includes a mark part indicating an arrangement state of the target parts.

With the X-ray generation target, it is possible to ascertain the arrangement state of the target parts using the mark part. Accordingly, it is possible to assemble the X-ray generation target, for example, in a state in which the longitudinal direction of the target parts is set to a prescribed direction. That is, it is possible to set the arrangement state of the plurality of target parts to a desired arrangement state.

The mark part may indicate a longitudinal direction or a transverse direction of the target parts. In this case, it is possible to set the longitudinal direction or the transverse direction of the plurality of target parts to a desired direction.

The target part holder may include a profile with a shape including an edge extending in the longitudinal direction or the transverse direction of the target parts in a view in a direction facing a burial surface in which the target parts are buried as the mark part. In this case, it is possible to ascertain the longitudinal direction or the transverse direction of the target parts based on the edge of the profile of the target part holder.

The target part holder may include a profile with a truncated-circle shape in the view in the direction facing the burial surface in which the target parts are buried as the mark part. In this case, it is possible to easily ascertain the longitudinal direction or the transverse direction of the target parts based on the truncated-circle shape of the profile of the target part holder.

The target part holder may include a profile with a polygonal shape in the view in the direction facing the burial surface in which the target parts are buried as the mark part. In this case, it is possible to easily ascertain the longitudinal direction or the transverse direction of the target parts based on a rectangular shape of the profile of the target part holder.

The target part holder may include a recess or a protrusion as the mark part. In this case, it is possible to ascertain the arrangement state of the target parts based on the recess or the protrusion of the target part holder.

The recess or the protrusion may include an edge extending in the longitudinal direction or the transverse direction of the target parts in the view in the direction facing the burial surface of the target part holder in which the target parts are buried. In this case, it is possible to easily ascertain the longitudinal direction or the transverse direction of the target parts as the arrangement state of the target parts from the edge of the recess or the protrusion of the target part holder.

The target parts may be formed continuously as a unified body in the longitudinal direction of the target parts, or the target parts may be formed discretely in the longitudinal direction of the target parts. In this case, it is possible to obtain X-rays under different irradiation conditions by configuring the long target parts in different forms.

An X-ray generator according to another aspect of the present disclosure includes: an electron gun emitting an electron beam; the X-ray generation target; and a support member supporting the X-ray generation target.

With the X-ray generator, since the X-ray generation target is provided, it is possible to set the arrangement state of the plurality of target parts to a desired arrangement state.

An X-ray generator according to another aspect of the present disclosure includes: an electron gun emitting an electron beam; the X-ray generation target; a support member supporting the X-ray generation target; and an irradiation area switching unit configured to switch an area irradiated with the electron beam between a first irradiation area and a second irradiation area in a view in a direction facing a burial surface of the target part holder in which the target parts are buried. The first irradiation area is an area including the plurality of target parts in the target part holder in the view in the direction facing the burial surface. The second irradiation area is an area including the inside and outside of the target parts over the edge in the view in the direction facing the burial surface.

With the X-ray generator, since the X-ray generation target is provided, it is possible to set the arrangement state of the plurality of target parts to a desired arrangement state. When the second irradiation area is irradiated with an electron beam, the edge of the profile of the target part holder appears as a boundary in an image acquired by detecting X-rays emitted from the X-ray generator in comparison with a case in which the first irradiation area is irradiated with an electron beam. Accordingly, it is also possible to ascertain the arrangement state of the target parts based on the boundary in the image.

The support member may include a recess into which the target part holder is fitted, and a profile of the target part holder which is the mark part may constitute a positioning part used to perform positioning such that the longitudinal direction or the transverse direction of the target parts with respect to the support member matches a desired direction. In this case, it is possible to ascertain the arrangement state of the target parts using the mark part and to perform positioning such that the longitudinal direction or the transverse direction of the target parts with respect to the support member is set to a desired direction.

The X-ray generator may further include: a housing accommodating at least a part of the electron gun, the X-ray generation target, and the support member; and an X-ray exit window that is provided in the housing and through which X-rays generated from the target parts exit to the outside of the housing. In this case, it is possible to provide an X-ray generator which can be handled in a state in which the arrangement state of the plurality of target parts is set to a desired arrangement state.

An X-ray imaging system according to another aspect of the present disclosure includes: the X-ray generator; an X-ray detector detecting X-rays emitted from the X-ray generator and passing through an object which is an imaging subject; a phase grating provided between the X-ray generator and the X-ray detector; and an absorption grating provided between the phase grating and the X-ray detector.

With the X-ray imaging system, since the X-ray generator is provided, it is possible to set the arrangement state of the plurality of target parts to a desired arrangement state.

### Advantageous Effects of Invention

According to the present disclosure, it is possible to provide an X-ray generation target, an X-ray generator, and an X-ray imaging system in which an arrangement state of a plurality of target parts in the X-ray generator can be set to a desired arrangement state.

### Brief Description of Drawings

FIG. 1 is a diagram schematically illustrating a configuration of an X-ray imaging system according to an embodiment.
FIG. 2 is a sectional view schematically illustrating an X-ray generator illustrated in FIG. 1.
FIG. 3 is a perspective view illustrating a support member and a heat dissipation part illustrated in FIG. 2.
FIG. 4(a) is a plan view illustrating an X-ray generation target, FIG. 4(b) is an enlarged view of the inside of a dotted frame in FIG. 4(a), and FIG. 4(c) is a sectional view taken along line A-A in FIG. 4(a).
FIG. 5(a) is a plan view illustrating an example of a method of manufacturing the X-ray generation target illustrated in FIG. 4(a), FIG. 5(b) is a plan view illustration subsequent to FIG. 5(a), FIG. 5(c) is a plan view illustration subsequent to FIG. 5(b), FIG. 5(d) is a plan view illustration subsequent to FIG. 5(c), FIG. 5(e) is a plan view illustration subsequent to FIG. 5(d), and FIG. 5(f) is a plan view illustration subsequent to FIG. 5(e).
FIG. 6(a) is a plan view illustrating a first irradiation area, and FIG. 6(b) is a diagram illustrating an image that is detected by an X-ray detector when an irradiation area is the first irradiation area.
FIG. 7(a) is a plan view illustrating a second irradiation area, and FIG. 7(b) is a diagram illustrating an image that is detected by the X-ray detector when an irradiation area is the second irradiation area.
FIG. 8(a) is a plan view illustrating a target part holding plate according to a modified example, FIG. 8(b) is a plan view illustrating a target part holding plate according to a modified example, FIG. 8(c) is a plan view illustrating a target part holding plate according to a modified example, and FIG. 8(d) is a plan view illustrating a target part holding plate according to a modified example.
FIG. 9 is a plan view schematically illustrating an X-ray generation target according to a modified example.
FIG. 10(a) is a plan view illustrating an example of a method of manufacturing the X-ray generation target illustrated in FIG. 9, FIG. 10(b) is a plan view illustration subsequent to FIG. 10(a), FIG. 10(c) is a plan view illustration subsequent to FIG. 10(b), FIG. 10(d) is a plan view illustration subsequent to FIG. 10(c), FIG. 10(e) is a plan view illustration subsequent to FIG. 10(d), and FIG. 10(f) is a plan view illustration subsequent to FIG. 10(e).
FIG. 11 is a plan view schematically illustrating an X-ray generation target according to a modified example.
FIG. 12 is a sectional view schematically illustrating an X-ray generator according to a modified example.
FIG. 13 is a sectional view schematically illustrating an X-ray generator according to a modified example.

### Description of Embodiments

Hereinafter, an embodiment of an X-ray generator according to an aspect of the present disclosure will be described in detail.

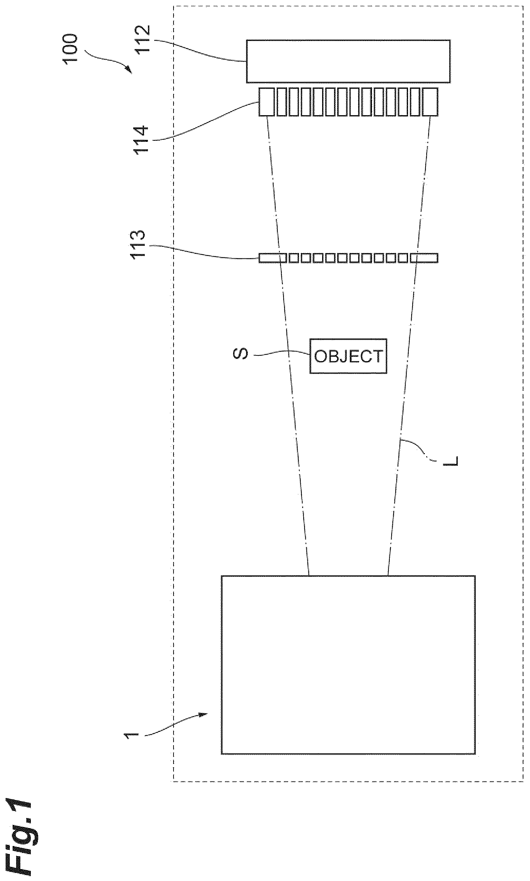

FIG. 1 is a diagram schematically illustrating a configuration of an X-ray imaging system 100 according to an embodiment. As illustrated in FIG. 1, the X-ray imaging system 100 is an X-ray phase imaging system that can generate grayscale (contrast) of an image from differences in intensity and phases of X-rays L passing through an object S which is an imaging subject and acquire an X-ray image, a phase differential image, and a small-angle scattering image of absorption contrast. The X-ray imaging system 100 is, for example, a system that is used for noninvasive inspection of an object S. The X-ray imaging system 100 is a system that can perform imaging using a Talbot-Lau interferometer system.

The X-ray imaging system 100 includes, an X-ray generator 1, an X-ray detector 112, a phase grating 113, and an absorption grating 114. The X-ray generator 1 is an X-ray source emitting X-rays L. The X-ray detector 112 detects X-rays L emitted from the X-ray generator 1 and passing through the object S and acquires an image. The phase grating 113 is a grating that is disposed between the X-ray generator 1 and the X-ray detector 112, and is disposed between the object S and the X-ray detector 112 in this embodiment. The phase grating 113 may be disposed in front of the object S or between the X-ray generator 1 and the object S. The phase grating 113 includes a plurality of slits which are arranged at uniform intervals. The phase grating 113 forms a self-image by causing spherical waves diffracted by the slits to interfere with each other (a Talbot effect). The absorption grating 114 is a grating that is disposed between the phase grating 113 and the X-ray detector 112. The absorption grating 114 includes a plurality of slits arranged in a cycle corresponding to the phase grating 113.

In the X-ray imaging system 100, X-rays L emitted from the X-ray generator 1 and passing through the phase grating 113 and the absorption grating 114 form moire fringes, and the moire fringes are detected by the X-ray detector 112. When there is no object S, the moire fringes are linearly arranged at equal intervals. On the other hand, when there is an object S, the fringes of X-rays L passing through the phase grating 113 are deformed by the object S, and the moire fringes are also deformed. The amount of deformation is phase information (corresponding to a phase shift). The moire fringes including deformation can be detected by the X-ray detector 112. Deformation of the fringes of X-rays L passing through the phase grating 113 is small and thus cannot be satisfactorily detected by the X-ray detector 112, but the deformation is enlarged to a size which can be detected as deformation of the moire fringes by providing the absorption grating 114.

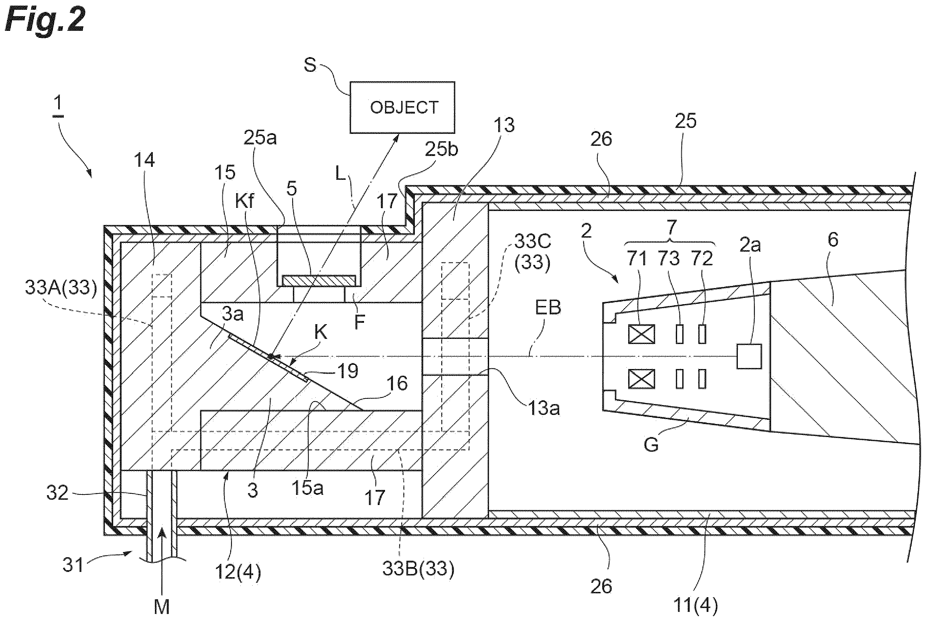

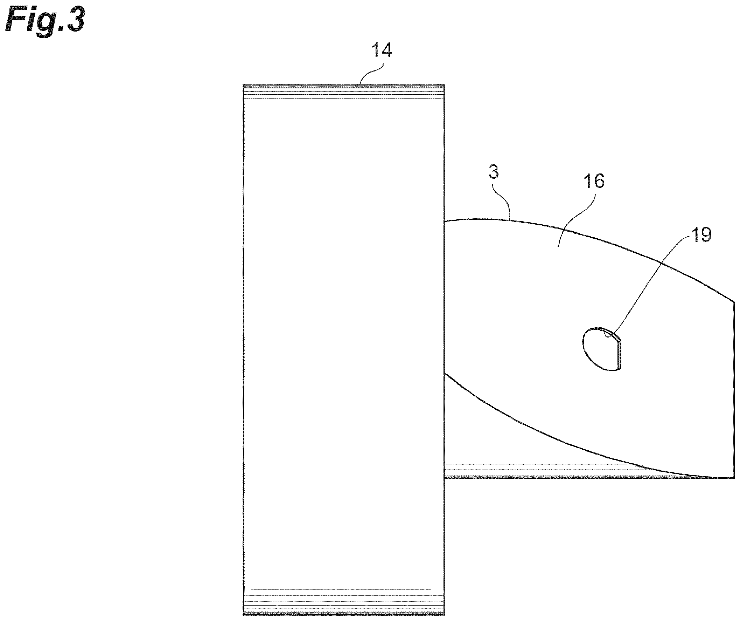

FIG. 2 is a sectional view schematically illustrating the X-ray generator 1 illustrated in FIG. 1. FIG. 3 is a perspective view illustrating a support member 3 and a heat dissipation part 14 in FIG. 2. As illustrated in FIG. 2, the X-ray generator 1 includes an electron gun 2, an X-ray generation target K, a support member 3, a housing 4, an X-ray exit window 5, and a cooling mechanism 31. The X-ray generator 1 is a reflection type X-ray tube that takes out X-rays L emitted in a direction crossing an incidence direction of an electron beam EB on the X-ray generation target K.

The electron gun 2 emits an electron beam EB. The electron gun 2 is, for example, a part that generates and emits an electron beam EB having energy of about from several keV to several hundreds of keV. The electron gun 2 includes a filament 2a, a grid G, an irradiation area switching unit 7 which will be described later, and an internal wire electrically connected thereto. The filament 2a constitutes a cathode. The filament 2a is an electron emission member emitting electrons which become an electron beam EB and is formed of, for example, a material including tungsten as a major component. The grid G is an electric field formation member deriving electrons and curbing diffusion of the electrons and is disposed to cover the filament 2a and the irradiation area switching unit 7.

A base part 6 holding the electron gun 2 is formed of, for example, an insulating material such as ceramic or epoxy. A high breakdown voltage type connector (not illustrated) that is supplied with a source voltage of about from several kV to several hundreds of kV from the outside of the X-ray generator 1 is attached to an end of the base part 6. The internal wire connected to the filament 2a or the like is connected to the high breakdown voltage type connector via the inside of the base part 6.

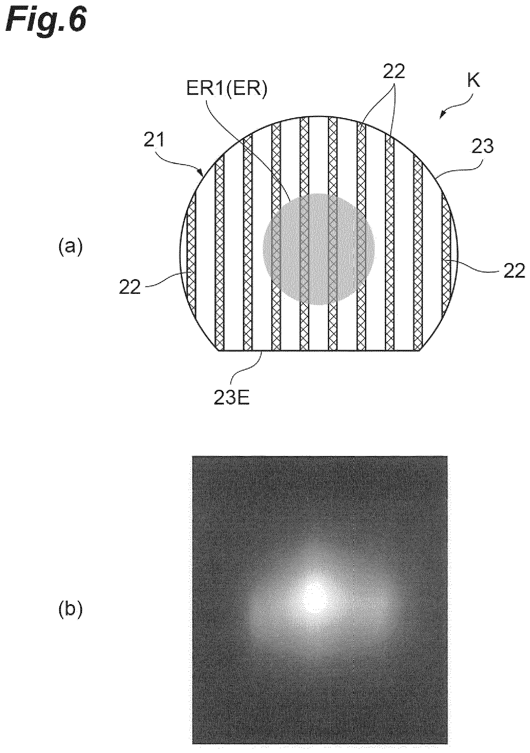

The filament 2a is heated to a high temperature with supply of a current from an external power supply and emits electrons with supply of a negative high voltage of about from - several kV to - several hundreds of kV. Electrons emitted from the filament 2a exit as an electron beam EB from a hole or a slit formed in a part of the grid G. A negative high voltage is applied to the filament 2a, and the housing 4 and the X-ray generation target K (and the support member 3) serving as an anode have a ground potential (an earth potential). Accordingly, the electron beam EB emitted from the electron gun 2 is incident on the X-ray generation target K in a state in which the electron beam is accelerated due to a potential difference between the filament 2a and the X-ray generation target K. In the X-ray generation target K, X-rays L are generated in response to the incident electron beam EB. The size of the electron beam EB (a beam size) at an incidence position on the X-ray generation target K, that is, an irradiation area ER of the electron beam EB (see FIG. 6(a)), is, for example, about φ 1 mm.

The housing 4 accommodates the electron gun 2, the X-ray generation target K, and the support member 3. The housing 4 includes an electron gun accommodation section 11 accommodating the electron gun 2 and a support member accommodation section 12 accommodating the support member 3. The housing 4 constitutes a substantially cylindrical vacuum container as a whole by air-tightly coupling the electron gun accommodation section 11 and the support member accommodation section 12. The electron gun accommodation section 11 is formed in a hollow cylindrical shape out of a metallic material such as stainless steel and is disposed to surround the electron gun 2. A fore-end part (an exit side of an electron beam EB) of the electron gun accommodation section 11 is air-tightly coupled to an aperture part 13 which will be described later of the support member accommodation section 12. For example, an aperture with a circular sectional shape is provided in a base-end part of the electron gun accommodation section 11, and a lid part in which the aforementioned high breakdown voltage type connector is provided is air-tightly coupled to the aperture.

The support member accommodation section 12 is formed of, for example, a metallic material with excellent electrical conductivity and thermal conductivity such as copper. In this embodiment, the support member accommodation section 12 includes an aperture part 13 through which an electron beam EB from the electron gun 2 is introduced to the X-ray generation target K, a heat dissipation part 14 thermally coupled to a base-end part 3a of the support member 3, and a window holding part 15 surrounding the support member 3 and holding the X-ray exit window 5. The window holding part 15 is formed in a hollow cylindrical shape, and the aperture part 13 and the heat dissipation part 14 are formed in a disc shape. The support member accommodation section 12 is formed in a cylindrical shape as a whole to surround the support member 3 by air-tightly coupling the aperture part 13 to one end (the electron gun 2 side) of the window holding part 15 and air-tightly coupling the heat dissipation part 14 to the other end (the opposite side to the electron gun 2) of the window holding part 15.

The aperture part 13 has, for example, a disc shape having substantially the same outer diameter as the outer diameter of the electron gun accommodation section 11. An aperture (13a) with a circular sectional shape penetrating the aperture part in a thickness direction thereof is provided substantially at the center of the aperture part 13. An electron beam EB emitted from the electron gun 2 is introduced into the support member accommodation section 12 via the aperture 13a.

As illustrated in FIGS. 2 and 3, the heat dissipation part 14 has, for example, a disc shape having a diameter slightly larger than that of the aperture part 13. In the heat dissipation part 14, the support member 3 protruding toward the aperture part 13 and located in the window holding part 15 is provided on the opposite surface side to the aperture part 13. The support member 3 supports the X-ray generation target K and is formed of, for example, a material with high thermal conductivity such as copper. Here, the support member 3 and the heat dissipation part 14 are formed as a unified body, but they may be separate bodies. One surface of the support member 3 has an arc shape corresponding to an inner circumferential surface 15a of the window holding part 15 and is air-tightly coupled to the inner circumferential surface 15a. Accordingly, the support member 3 protruding toward the aperture part 13 is thermally coupled to the window holding part 15.

The X-ray generation target K is disposed on a target support surface 16 on the other surface side of the support member 3 such that target parts 22 face the electron gun 2 with a predetermined tilt angle with respect to an emission axis of an electron beam EB. Specifically, a recess 19 is formed in the target support surface 16, and the X-ray generation target K is fitted into the recess 19. The recess 19 is a recess with a shape corresponding to the profile of the X-ray generation target K. Here, the recess 19 has a depth corresponding to the thickness of a target part holding plate (a target part holder) 21 which will be described later and has a shape corresponding to the profile (a truncated-circle shape) of the target part holding plate 21 which will be described later in a view in a direction opposite to the target support surface 16.

A rear surface and a side surface of the X-ray generation target K come into an inner surface of the recess 19 directly or via a bonding member with high thermal conductivity. The target support surface 16 and a surface Kf of the X-ray generation target K fitted into the recess 19 which is an electron incidence side are flush with each other. That is, the target support surface 16 is disposed on the same plane as the surface Kf of the X-ray generation target K.

As illustrated in FIG. 2, the window holding part 15 has a cylindrical shape with the same diameter as that of the heat dissipation part 14. In the window holding part 15, a fixing part F to which the X-ray exit window 5 is fixed is provided in a circumferential wall part 17 facing the target support surface 16. A rectangular aperture smaller than the X-ray exit window 5 is formed in the fixing part F. A circumferential part of the X-ray exit window 5 is bonded to an edge part of the aperture by soldering or the like, and thus the aperture is air-tightly sealed by the X-ray exit window 5. The X-ray exit window 5 fixed to the fixing part F is disposed to face the X-ray generation target K at a predetermined tilt angle.

A case 25 covering the housing 4 is provided in the housing 4. The case 25 is formed in a substantially rectangular parallelepiped shape out of, for example, a conductive material such as metal. In the case 25, an aperture 25a with the same shape as the planar shape of the fixing part F is provided at a position corresponding to the fixing part F of the X-ray exit window 5. An X-ray shielding member 26 is disposed on the inner surface side of the case 25 except for the position of the aperture 25a. The X-ray shielding member 26 is formed of a material with high X-ray shielding capability (for example, a heavy metal material such as lead) and is disposed between the case 25 and the housing 4. Accordingly, unnecessary leakage of X-rays L is curbed, the case 25 and the housing 4 are electrically connected, and the ground potential of the X-ray generator 1 is stably secured.

The cooling mechanism 31 cools the support member accommodation section 12. The cooling mechanism 31 includes a connection pipe 32 for introducing and discharging a coolant and a cooling flow channel 33 for allowing a coolant M to circulate in the wall part of the support member accommodation section 12. The cooling flow channel 33 is a through-hole formed inside of the wall part of the support member accommodation section 12 and is disposed in at least the heat dissipation part 14 and the aperture part 13. The cooling flow channel 33 includes a first cooling flow channel 33A provided in the heat dissipation part 14, a second cooling flow channel 33B provided in the window holding part 15, and a third cooling flow channel 33C provided in the aperture part 13. For example, water or ethylene glycol is used as the coolant M.

The connection pipe 32 is connected to the cooling flow channel 33 and protrudes externally from the case 25. A pair of connection pipes 32 is provided, one connection pipe 32 serves as a pipe for introducing the coolant M into the cooling flow channel 33 from an external circulation device, and the other connection pipe 32 serves as a pipe for discharging the coolant M circulating in the cooling flow channel 33 to the external circulation device. In the cooling mechanism 31, a coolant introduced via the one connection pipe 32 flows in the first cooling flow channel 33A and is discharged from the other connection pipe 32. A part of the coolant introduced into the first cooling flow channel 33A via the one connection pipe 32 branches from the first cooling flow channel 33A, flows in the second cooling flow channel 33B, and is introduced into the third cooling flow channel 33C. The coolant flowing in the third cooling flow channel 33C flows in the second cooling flow channel 33B, returns to the first cooling flow channel 33A, and is discharged from the other connection pipe 32.

In the X-ray generator 1 having the aforementioned configuration, an electron beam EB is incident on the target parts 22 which will be described later on the X-ray generation target K, and X-rays L generated from the X-ray generation target K in response to incidence of the electron beam EB is transmitted by the X-ray exit window 5 and is taken out externally from the X-ray generator 1.

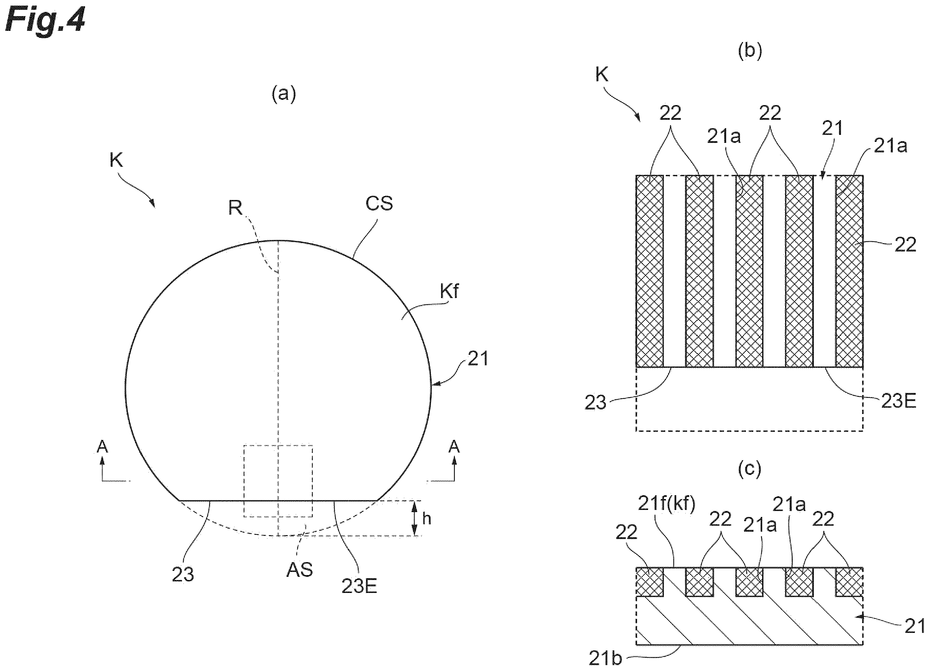

FIG. 4(a) is a plan view illustrating the X-ray generation target K. FIG. 4(b) is an enlarged view of the inside of a dotted frame in FIG. 4(a). FIG. 4(c) is a sectional view taken along line A-A in FIG. 4(a). In the sectional view illustrated in FIG. 4(c), the inside of the dotted frame in FIG. 4(a) is illustrated. As illustrated in FIGS. 4(a), 4(b), and 4(c), the X-ray generation target K includes a plurality of long target parts 22 generating X-rays L in response to incidence of an electron beam EB and a target part holding plate 21 in which the plurality of target parts 22 is buried to be parallel with each other.

The target part holding plate 21 is a panel-shaped member formed of a material with a higher thermal conductivity than the material of the target parts 22 such as single-crystal diamond, polycrystalline diamond, or copper. The thermal conductivity of the material of the target part holding plate 21 may be equal to or higher than the thermal conductivity of the material of the support member 3. The target part holding plate 21 includes a surface (a burial surface) 21f which is an electron beam incidence surface and a rear surface 21b which is opposite to the front surface 21f and which serves as a physical and thermal connection part to the support member 3. A plurality of bottomed grooves 21a with a rectangular sectional shape are formed in the front surface 21f of the target part holding plate 21 over the entire surface thereof. The grooves 21a extend linearly in parallel to each other. Both ends of each groove 21a reach the edge of the front surface 21f and are open ends. That is, each groove 21a is a long groove formed to linearly connect the edge and the edge of the front surface 21f and to extend in parallel with a segment R indicating the diameter in a circular shape CS of the target part holding plate 21 which will be described later.

The target parts 22 are formed of, for example, metal such as tungsten. The target parts 22 are provided in the target part holding plate 21 to fill the grooves 21a. The target parts 22 are linear target parts extending linearly in parallel to each other and each thereof has a long rectangular parallelepiped shape. That is, each target part 22 is continuously as a unified body in the longitudinal direction. An area of the X-ray generation target K in which the target parts 22 are arranged is larger than the size of the electron beam EB (a beam size), that is, an irradiation area ER of the electron beam EB (see FIG. 6(a)).

In this embodiment, the target part holding plate 21 includes a mark part 23 indicating the longitudinal direction of the target parts 22. Specifically, the target part holding plate 21 includes a profile with a shape including an edge 23E in a view in the direction facing the front surface 21f of the target part holding plate 21, that is, a thickness direction of the target part holding plate 21 (hereinafter simply referred to as a "thickness direction"), as the mark part 23. Here, the target part holding plate 21 has a profile with a truncated-circle shape in the view in the thickness direction and includes the profile with a truncated-circle shape as the mark part 23. The edge 23E extends in the transverse direction of the target parts 22 in the view in the thickness direction. The truncated-circle shape is a shape in which a part of a circle is truncated. The truncated-circle shape is a shape in which an arc shape AS is removed from a circular shape CS (see FIG. 4(a)). The edge 23E extends in a direction perpendicular to the segment R indicating the diameter of the circular shape CS. The profile of the target part holding plate 21 which is the mark part 23 constitutes a positioning part positioning the longitudinal direction of the target parts 22 with respect to the support member 3.

In this embodiment, the diameter of the circular shape CS of the target part holding plate 21 ranges from 3 mmφ to 12 mmφ, and the thickness of the target part holding plate 21 ranges from 0.1 mm to 3 mm. The pitch of the target parts 22 (an inter-center distance between neighboring target parts 22) ranges from 5 µm to 20 µm, the width of the target parts 22 ranges from 2 µm to 10 µm, and the depth of the target parts 22 ranges from 5 µm to 30 µm. The truncated-circle shape of the target part holding plate 21 has a degree in which the height h of the arc shape AS (see FIG. 4(a)) is equal to or less than a radius of the circular shape CS. Each target part 22 has a long shape extending in parallel to the segment R indicating the diameter of the circular shape CS of the target part holding plate 21. In other words, the longitudinal direction of the target parts 22 is the same as the direction in which the segment R indicating the diameter of the circular shape CS of the target part holding plate 21 extends.

As illustrated in FIGS. 2 and 3, the X-ray generation target K is assembled into the recess 19 of the target support surface 16 of the support member 3 while ascertaining and adjusting an angle in the longitudinal direction (an angle in the rotating direction of thickness-direction rotation) of the target parts 22 using the mark part 23. Specifically, the direction of the target part holding plate 21 is adjusted such that the truncated-circle shape of the target part holding plate 21 matches the truncated-circle shape of the recess 19, and thus the longitudinal direction of the target parts 22 of the X-ray generation target K is set to a desired direction (a predetermined direction). The X-ray generation target K is fitted and bonded to the recess 19 via a bonding member such as silver solder. As a result, the X-ray generation target K is positioned in a state in which the longitudinal direction of the target parts 22 is set to the desired direction and is buried such that the front surface Kf is flush with the target support surface 16.

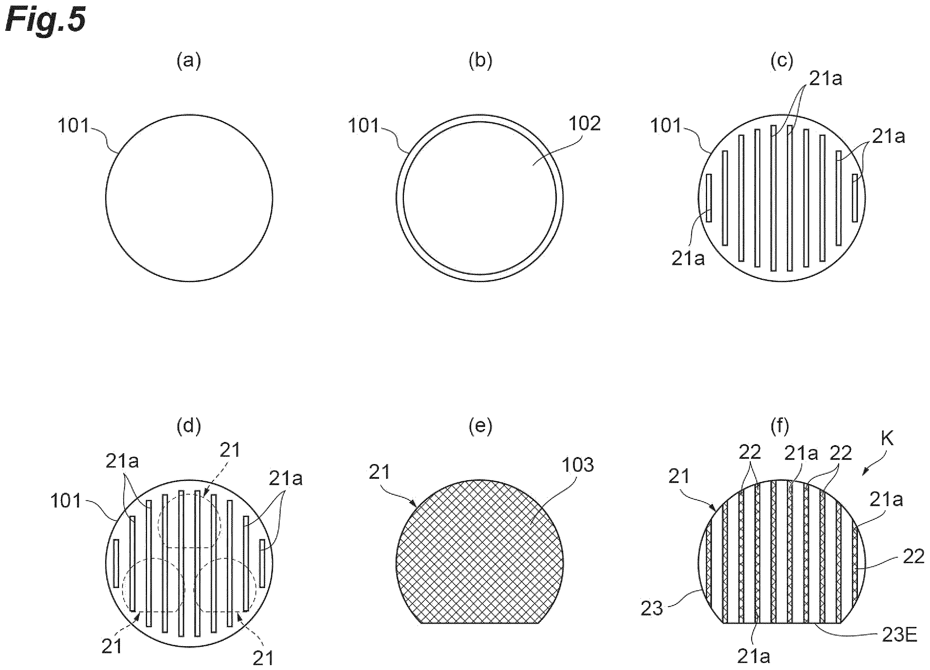

FIGS. 5(a) to 5(f) are plan views illustrating an example of a method of manufacturing the X-ray generation target K. In FIGS. 5(a) to 5(f), dimensional ratios in the drawings do not necessarily match those of actual objects to be described. As illustrated in FIG. 5(a), a disc-shaped substrate 101 is prepared. As illustrated in FIG. 5(b), a resist 102 is applied to the surface of the substrate 101, and a periodical structure corresponding to the grooves 21a is transferred to the substrate 101. As illustrated in FIG. 5(c), a plurality of grooves 21a is formed in the surface of the substrate 101, for example, using inductive coupled plasma-reactive ion etching (ICP-RIE).

As illustrated in FIG. 5(d), a plurality of target part holding plates 21 is cut out from the substrate 101. As illustrated in FIG. 5(e), the surface of the target part holding plate 21 is covered with a metal 103, for example, using chemical vapor deposition (CVD) such that the metal 103 is buried in the plurality of grooves 21a of the target part holding plate 21. As illustrated in FIG. 5(f), surplus parts (mainly parts not buried in the grooves 21a) of the metal 103 attached to the surface of the target part holding plate 21 are removed using chemical mechanical polishing (CMP) or the like to form the target parts 22. A substrate protecting film (not illustrated) of titanium or the like is formed on the front surface 21f of the target part holding plate 21. In this way, the X-ray generation target K is completed.

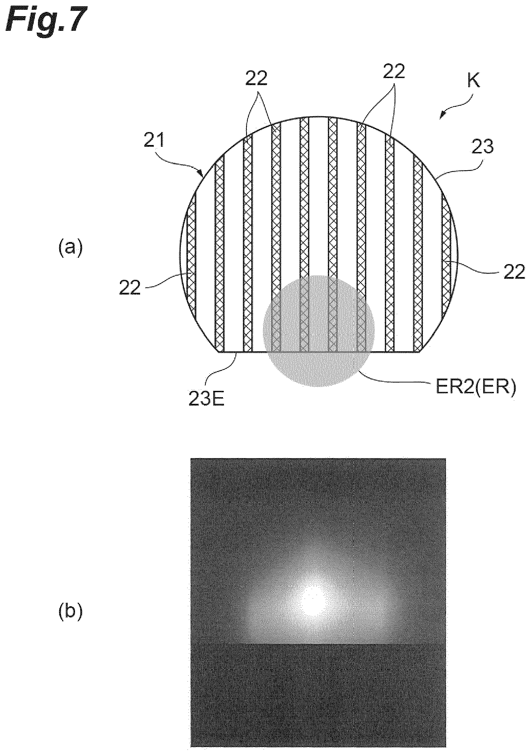

Referring back to FIG. 2, the X-ray generator 1 includes an irradiation area switching unit 7 switching the irradiation area ER irradiated with an electron beam EB between a first irradiation area ER1 (see FIG. 6(a)) and a second irradiation area ER2 (see FIG. 7(a)) in a view in the thickness direction. The first irradiation area ER1 is an area including a plurality of target parts 22 in the target part holding plate 21 in a view in the thickness direction (see FIG. 6(a)). The second irradiation area ER2 is an area including the inside and the outside of the target part holding plate 21 over the edge 23E of the profile of the target part holding plate 21 in the view in the thickness direction. In other words, the second irradiation area ER2 is an area over the X-ray generation target K and the support member 3 in the view in the thickness direction. The irradiation area ER irradiated with an electron beam EB is about 1 mmφ.

The irradiation area switching unit 7 is provided in the electron gun 2. The irradiation area switching unit 7 includes a deflecting coil 71, a first electrode 72, and a second electrode 73. The deflecting coil 71 adjusts a position of an electron beam EB in a direction perpendicular to an exit axis of the electron beam EB. The first electrode 72 adjusts an amount of electrons of the electron beam EB emitted from the filament 2a. The first electrode 72 is disposed between the filament 2a and the deflecting coil 71. The second electrode 73 adjusts a beam size of an electron beam EB by changing a voltage thereof. The second electrode 73 is disposed between the first electrode 72 and the deflecting coil 71.

With the X-ray generation target K, the X-ray generator 1, and the X-ray imaging system 100 which are described above, it is possible to ascertain the longitudinal direction of the target parts 22 using the mark part 23. Accordingly, it is possible to assemble the X-ray generation target K, for example, in a state in which the longitudinal direction of the target parts 22 is reliably set to a desired direction. That is, it is possible to set the longitudinal direction of the plurality of target parts 22 to a desired direction. That is, it is possible to set the arrangement state of the plurality of target parts 22 to a desired arrangement state. Accordingly, since X-rays satisfying a desired irradiation condition in the X-ray generator 1 can be obtained, it is possible to easily realize high alignment accuracy for optical system alignment of the X-ray imaging system 100. When the arrangement state (for example, the longitudinal direction) of the plurality of target parts 22 can be set to a desired arrangement state, it causes securement of accuracy of optical system alignment and thus causes simplification of optical system alignment.

The target part holding plate 21 includes a profile with a shape including the edge 23E extending in the transverse direction of the target parts 22 in a view in the direction facing the front surface 21f (thickness direction) as the mark part 23. In this case, it is possible to ascertain the longitudinal direction of the target parts 22 based on the edge 23E of the profile of the target part holding plate 21.

The target part holding plate 21 includes a profile with a truncated-circle shape in the view in the direction facing the front surface 21f (the thickness direction) as the mark part 23. In this case, it is possible to easily ascertain the longitudinal direction of the target parts 22 based on the truncated-circle shape of the profile of the target part holding plate 21.

In the X-ray generator 1, the irradiation area ER irradiated with an electron beam EB is switched between the first irradiation area ER1 and the second irradiation area ER2. The first irradiation area ER1 is an area including a plurality of target parts 22 in the target part holding plate 21 (see FIG. 6(a)), and the second irradiation area ER2 is an area including the inside and the outside of the target part holding plate 21 over the edge 23E (see FIG. 7(a)). As illustrated in FIGS. 6(b) and 7(b), when the second irradiation area ER2 is irradiated with an electron beam EB, the edge 23E appears as a boundary in an image acquired by the X-ray detector 112 in comparison with a case in which the first irradiation area ER1 is irradiated with an electron beam EB. Accordingly, it is also possible to ascertain the arrangement state (the longitudinal direction) of the target parts 22 based on the boundary in the image.

In the X-ray generator 1, the profile of the target part holding plate 21 which is the mark part 23 constitutes a positioning part performing positioning such that the longitudinal direction of the target parts 22 with respect to the support member 3 is set to a desired direction. Accordingly, it is possible to ascertain the longitudinal direction of the target parts 22 using the mark part 23 and to perform positioning such that the longitudinal direction of the target parts 22 with respect to the support member 3 is set to a desired direction.

The X-ray generator 1 is a sealed reflection type device. In the reflection type X-ray generator 1, since there is more difficulty in ascertaining the arrangement state of the target parts 22 from the outside of the housing 4 in comparison with a transmission type, the aforementioned effect capable of setting the arrangement state of the plurality of target parts 22 to a desired arrangement state becomes more marked.

The present disclosure is not limited to the embodiment.

In the aforementioned embodiment, the profile with the truncated-circle shape of the target part holding plate 21 is included as the mark part 23, but the mark part 23 is not particularly limited and various elements may be used as long as they can indicate the arrangement state of the target parts 22.

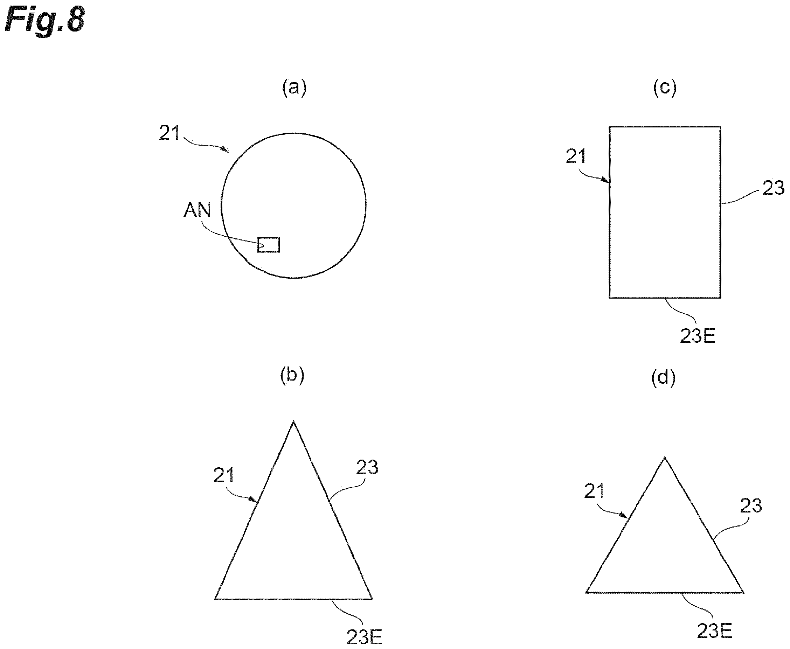

For example, as illustrated in FIG. 8(a), the target part holding plate 21 may have a disc shape and include a hole AN (a recess) penetrating the target part holding plate 21 in the thickness direction thereof as the mark part 23. The hole AN has a rectangular shape in a view in the thickness direction. The hole AN includes an edge extending in the longitudinal direction of the target parts 22 and an edge extending in the transverse direction thereof in the view in the thickness direction. For example, the hole AN has a rectangular shape of 2 mm×1 mm. In this case, a circular recess 19 is formed in the target support surface 16 of the support member 3, and a rectangular protrusion corresponding to the shape of the hole AN is formed as a protrusion fitted into the hole AN in the recess 19.

Accordingly, it is possible to ascertain the arrangement state of the target parts 22 based on the hole AN of the target part holding plate 21. It is possible to easily ascertain the longitudinal direction of the target parts 22 based on the edge of the hole AN of the target part holding plate 21. The shape of the hole AN is not limited to a rectangular shape and may have various shapes. A hole with a shape including an edge extending in one of the longitudinal direction and the transverse direction of the target parts 22 may be formed instead of the hole AN. A groove or a protrusion may be formed instead of the hole AN. The shape of the hole AN and the arrangement state (the longitudinal direction) of the target parts 22 may be correlated and the arrangement state may be ascertained with the shape of the hole AN as a mark. The position of the hole AN in the target part holding plate 21 may be correlated with the arrangement state of the target parts 22, and the arrangement state may be ascertained with the position of the hole AN as a mark.

For example, as illustrated in FIGS. 8(b) to 8(d), the target part holding plate 21 may have a profile with a polygonal shape as the mark part 23 in the view in the thickness direction. For example, as illustrated in FIG. 8(b), the target part holding plate 21 may have a profile with an equilateral triangular shape including an edge 23E as the mark part 23 in the view in the thickness direction. In this case, a recess 19 with an equilateral triangular shape corresponding to the profile of the mark part 23 is formed in the target support surface 16 of the support member 3. Accordingly, it is possible to easily ascertain the arrangement state (the longitudinal direction) of the target parts 22 based on the equilateral triangular shape of the profile of the target part holding plate 21.

For example, as illustrated in FIG. 8(c), the target part holding plate 21 may have a profile with a rectangular shape including an edge 23E as the mark part 23 in the view in the thickness direction. In this case, a recess 19 with a rectangular shape corresponding to the profile of the mark part 23 is formed in the target support surface 16 of the support member 3. Accordingly, it is possible to easily ascertain the arrangement state (the longitudinal direction) of the target parts 22 based on the rectangular shape of the profile of the target part holding plate 21.

For example, as illustrated in FIG. 8(d), the target part holding plate 21 may have a profile with a regular triangular shape including an edge 23E as the mark part 23 in the view in the thickness direction. In this case, a recess 19 with a regular triangular shape corresponding to the profile of the mark part 23 is formed in the target support surface 16 of the support member 3. Accordingly, it is possible to easily ascertain the arrangement state (the longitudinal direction) of the target parts 22 based on the regular triangular shape of the profile of the target part holding plate 21.

In the embodiment, the edge 23E included in the mark part 23 extends in the transverse direction of the target parts 22 in the view in the thickness direction, but is not limited thereto. The edge 23E may extend in the longitudinal direction of the target parts 22 in the view in the thickness direction or may extend in a direction tilted by a predetermined angle with respect to the longitudinal direction or the transverse direction of the target parts 22.

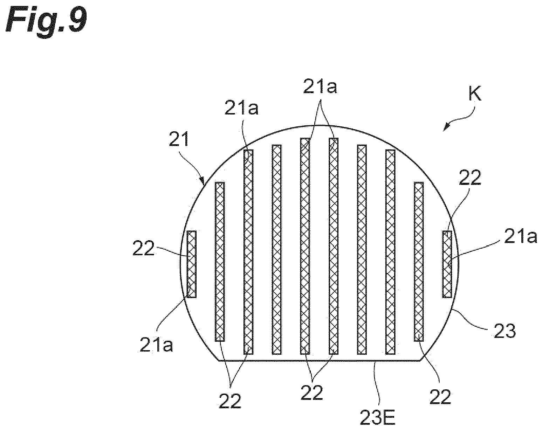

In the embodiment, both ends of a plurality of grooves 21a formed in the target part holding plate 21 reach the side surface 21s to form open ends and the target parts 22 reach the edge of the target part holding plate 21 (see FIG. 5(f)), but the present disclosure is not limited thereto. As illustrated in FIG. 9, both ends of each of the plurality of grooves 21a in the target part holding plate 21 do not reach the edge of the target part holding plate 21 (without forming open ends), but a configuration in which the target parts 22 do not reach the edges of the target part holding plate 21 may be employed.

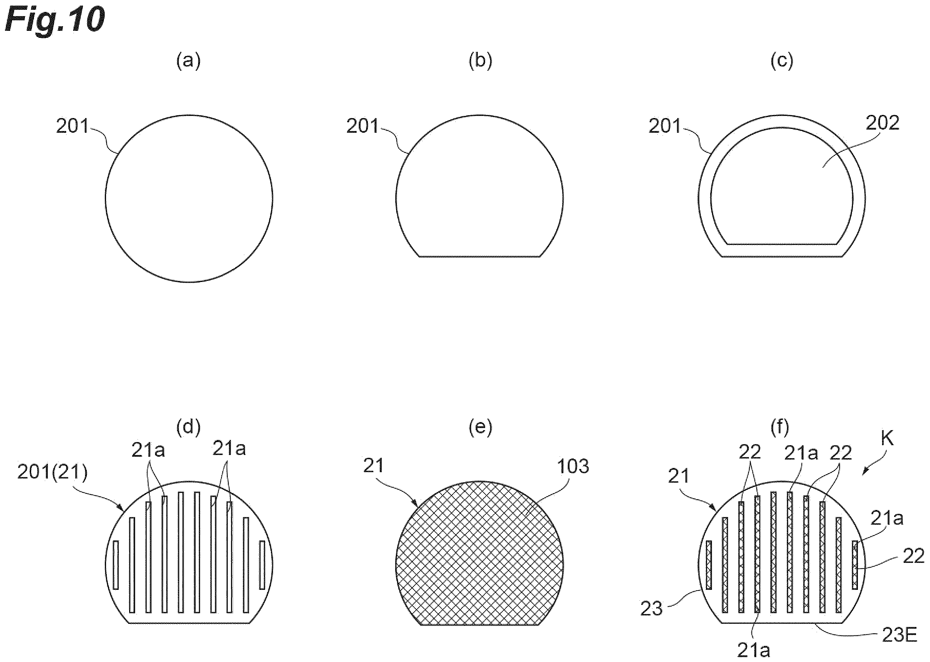

When the X-ray generation target K illustrated in FIG. 9 is manufactured, a disc-shaped substrate 201 is prepared as illustrated in FIG. 10(a). As illustrated in FIG. 10(b), a part with an arc shape of the substrate 201 is notched and cut out such that the profile of the substrate 201 has a truncated-circle shape. As illustrated in FIG. 10(c), a resist 202 is applied to the surface of the substrate 201, and a periodical structure corresponding to the grooves 21a is transferred to the substrate 201. As illustrated in FIG. 10(d), a plurality of grooves 21a is formed in the surface of the substrate 201, for example, using inductive coupled plasma-reactive ion etching, and thus the target part holding plate 21 is obtained.

As illustrated in FIG. 10(d), the surface of the target part holding plate 21 is covered with a metal 103, for example, using chemical vapor deposition such that the metal 103 is buried in the plurality of grooves 21a of the target part holding plate 21. As illustrated in FIG. 10(f), surplus parts (mainly parts not buried in the grooves 21a) of the metal 103 attached to the surface of the target part holding plate 21 are removed using chemical mechanical polishing or the like to form the target parts 22. A substrate protecting film (not illustrated) of titanium or the like is formed on the front surface 21f of the target part holding plate 21. In this way, the X-ray generation target K illustrated in FIG. 9 is completed.

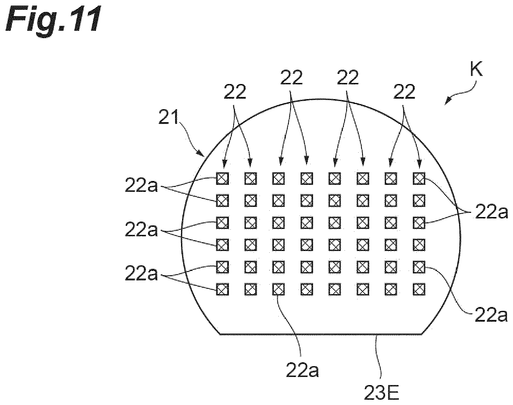

In the embodiment, the target parts 22 are formed continuously as a unified body in the longitudinal direction, but the present disclosure is not limited thereto. As illustrated in FIG. 11, the target parts 22 may be formed discretely in the longitudinal direction. More specifically, each target part 22 includes a plurality of divided target parts 22a which are linearly arranged in the longitudinal direction. The plurality of divided target parts 22a has the same shape in the view in the direction facing the front surface 21f (the burial surface) and is arranged at equal intervals in the longitudinal direction (the Y-axis direction). Since the plurality of target parts 22 is also arranged at equal intervals in the X-axis direction, the divided target parts 22a are arranged in a matrix shape. The interval between the divided target parts 22a in the longitudinal direction (the interval between the divided target parts 22a neighboring in the Y-axis direction) and the interval between the neighboring target parts 22 (the interval between the divided target parts 22a neighboring in the X-axis direction) may be the same or different from each other. By forming the long target parts 22 in different forms in this way, it is possible to obtain X-rays with different irradiation conditions.

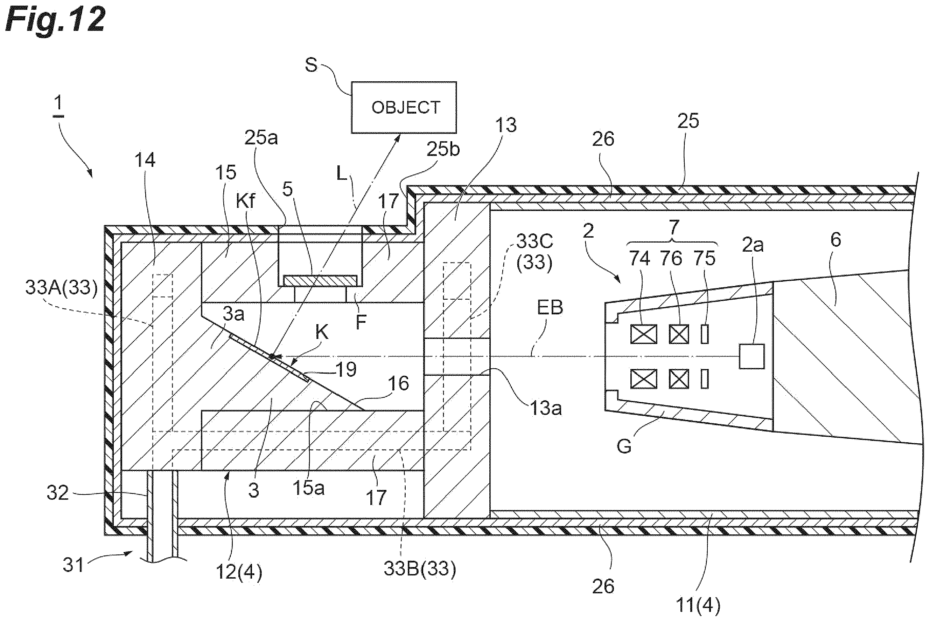

In the embodiment, the irradiation area switching unit 7 includes the deflecting coil 71, the first electrode 72, and the second electrode 73, but the configuration of the irradiation area switching unit 7 is not particularly limited. As illustrated in FIG. 12, the irradiation area switching unit 7 may include a focusing coil 74, a first electrode 75, and a deflecting coil 76. The focusing coil 74 adjusts the beam size of an electron beam EB by adjusting an amount of current flowing in the coil. The first electrode 75 adjusts an amount of electrons of the electron beam EB emitted from the filament 2a. The first electrode 75 is disposed between the filament 2a and the focusing coil 74. The deflecting coil 76 adjusts the position of the electron beam EB in a direction perpendicular to the emission axis of the electron beam EB. The deflecting coil 76 is disposed between the first electrode 75 and the focusing coil 74. The irradiation area switching unit 7 may not include, for example, at least one of the first electrodes 72 and 75, the second electrode 73, and the focusing coil 74 as long as it can switch the irradiation area ER.

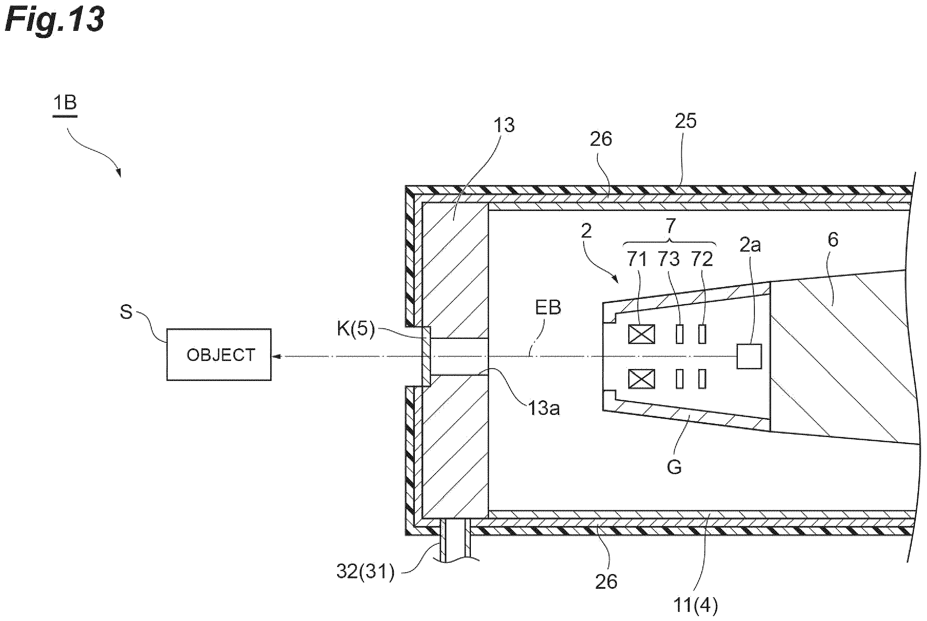

The embodiment includes the reflection type X-ray generator 1, but the present disclosure is not limited thereto. For example, as illustrated in FIG. 13, a transmission type X-ray generator 1B taking out X-rays L emitted in a direction based on an incidence direction of an electron beam EB on the X-ray generation target K such that the electron beam passes through the X-ray generation target K may be provided. In the X-ray generator 1B in the illustrated example, the X-ray generation target K also serving as the X-ray exit window 5 is disposed in the aperture part 13 such that the X-ray generation target K is perpendicular to the emission axis of the electron beam EB.

In the embodiment, the support member accommodation section 12 has a configuration in which the aperture part 13, the heat dissipation part 14, and the window holding part 15 are combined, but the configuration of the support member accommodation section 12 is not limited thereto. For example, the window holding part 15 incorporated into the aperture part 13 may be coupled to the heat dissipation part 14 to form the support member accommodation section 12, or the window holding part 15 incorporated into the heat dissipation part 14 may be coupled to the aperture part 13 to form the support member accommodation section 12. The mark part 23 may not explicitly indicate the longitudinal direction of the target parts 22 visually and intuitively, and has only to include information capable of determining the longitudinal direction of the target parts 22. The target parts 22 are provided to fill the bottomed grooves 21a, but the grooves 21a may be provided in a state in which they penetrate the target part holding plate 21 such that they reach from the front surface 21f to the rear surface 21b. The target parts 22 are held by the target part holding plate 21 which is a plane-shaped member, but may be held by a block-shaped holding member. A structure formed of a material different from that of the target part holding plate 21 may be provided as the mark part 23 on the target part holding plate 21.

In the embodiment and the modified examples, the longitudinal direction of a plurality of target parts 22 is ascertained, but the transverse direction of the plurality of target parts 22 may be ascertained. In the embodiment and the modified examples, the longitudinal direction of the plurality of target parts 22 is used as a desired direction, but the transverse direction of the plurality of target parts 22 may be used as a desired direction. It is possible to ascertain the longitudinal direction or the transverse direction of the target parts. In the embodiment, positioning is performed such that the longitudinal direction of the target parts 22 with respect to the support member 3 is the desired direction, but positioning may be performed such that the transverse direction of the target parts 22 with respect to the support member 3 is the desired direction. In the embodiment and the modified examples, indication of the longitudinal direction or the transverse direction may include direct indication of the longitudinal direction or the transverse direction and indirect indication of the longitudinal direction or the transverse direction, for example, by indicating an arrangement direction or the like. In the embodiment and the modified examples, the housing 4 has only to accommodate at least a part of the support member 3.

The configurations according to the embodiment and the modified examples are not limited to the aforementioned materials and shapes, and can employ various materials and shapes. The configurations according to the embodiment or the modified examples can be arbitrarily applied to the configurations of the other embodiment or modified examples. Some of the configurations in the embodiment or modified examples can be appropriately omitted without departing from the gist of an aspect of the present disclosure.

### Reference Signs List

1, 1B X-ray generator
2 Electron gun
3 Support member
4 Housing
5 X-ray exit window
7 Irradiation area switching unit
19 Recess
21 Target part holding plate (target parts holder)
22 Target part
23 Mark part (positioning part)
23E Side
100 X-ray imaging system
112 X-ray detector
113 Phase grating
114 Absorption grating
AN Hole
EB Electron beam
L X-ray
ER Irradiation area
ER1 First irradiation area
ER2 Second irradiation area
K X-ray generation target
S Object.

## Claims

1. An X-ray generation target comprising:
a plurality of long target parts generating X-rays in response to incidence of an electron beam; and
a target part holder in which the plurality of target parts are buried to be parallel to each other,
wherein the target part holder includes a mark part indicating an arrangement state of the target parts.

2. The X-ray generation target according to claim 1, wherein the mark part indicates a longitudinal direction or a transverse direction of the target parts.

3. The X-ray generation target according to claim 2, wherein the target part holder includes a profile with a shape including an edge extending in the longitudinal direction or the transverse direction of the target parts in a view in a direction facing a burial surface in which the target parts are buried as the mark part.

4. The X-ray generation target according to claim 3, wherein the target part holder includes a profile with a truncated-circle shape in the view in the direction facing the burial surface in which the target parts are buried as the mark part.

5. The X-ray generation target according to claim 3, wherein the target part holder includes a profile with a polygonal shape in the view in the direction facing the burial surface in which the target parts are buried as the mark part.

6. The X-ray generation target according to any one of claims 1 to 5, wherein the target part holder includes a recess or a protrusion as the mark part.

7. The X-ray generation target according to claim 6, wherein the recess or the protrusion includes an edge extending in the longitudinal direction or the transverse direction of the target parts in the view in the direction facing the burial surface of the target part holder in which the target parts are buried.

8. The X-ray generation target according to any one of claims 1 to 7, wherein the target parts are formed continuously as a unified body in a longitudinal direction of the target parts.

9. The X-ray generation target according to any one of claims 1 to 7, wherein the target parts are formed discretely in a longitudinal direction of the target parts.

10. An X-ray generator comprising:
an electron gun emitting an electron beam;
the X-ray generation target according to any one of claims 1 to 9; and
a support member supporting the X-ray generation target.

11. An X-ray generator comprising:
an electron gun emitting an electron beam;
the X-ray generation target according to any one of claims 3 to 5;
a support member supporting the X-ray generation target; and
an irradiation area switching unit configured to switch an area irradiated with the electron beam between a first irradiation area and a second irradiation area in a view in a direction facing a burial surface of the target part holder in which the target parts are buried,
wherein the first irradiation area is an area including the plurality of target parts in the target part holder in the view in the direction facing the burial surface, and
wherein the second irradiation area is an area including the inside and outside of the target parts over the edge in the view in the direction facing the burial surface.

12. The X-ray generator according to claim 10 or 11, wherein the support member includes a recess into which the target part holder is fitted, and
wherein a profile of the target part holder which is the mark part constitutes a positioning part used to perform positioning such that the longitudinal direction or the transverse direction of the target parts with respect to the support member matches a desired direction.

13. The X-ray generator according to any one of claims 10 to 12, further comprising:
a housing accommodating at least a part of the electron gun, the X-ray generation target, and the support member; and
an X-ray exit window that is provided in the housing and through which X-rays generated from the target parts exit to the outside of the housing.

14. An X-ray imaging system comprising:
the X-ray generator according to any one of claims 10 to 13;
an X-ray detector detecting X-rays emitted from the X-ray generator and passing through an object which is an imaging subject;
a phase grating provided between the X-ray generator and the X-ray detector; and
an absorption grating provided between the phase grating and the X-ray detector.
